# EUROPEAN PATENT APPLICATION

(11) **EP 4 606 402 A1**
(43) Date of publication of application: **27.08.2025**
(21) Application number: 24305291.7
(22) Date of filing: 22.02.2024
(51) Int. Cl.: A61M 5/28, A61M 5/32

(54) **PRE-FILLABLE INJECTION DEVICE WITH EXTENDABLE POST-INJECTION NEEDLE SHIELD**

(71) Applicant: Becton Dickinson France, 38800 Le Pont-de-Claix (FR); Becton Dickinson Holdings Pte. Ltd., Singapore 639461 (SG)
(72) Inventor: VARGEESE, Raja, 625009 Tamil Nadu (IN); SUNDARALINGAM, Mahesh Kumar, Puthalam, 629602 Tamil Nadu (IN); HARMOKO, Hendri, Singapore 650621 (SG); LI, Xin, Singapore 567700 (SG)
(74) Representative: Germain Maureau

(57) **Abstract**

Provided herein is a pre-fillable injection device that includes an end member having a fluid-filled reservoir therein that is formed by a depressible film. A port and hub are provided on a distal end of the end member to provide access to the reservoir. A double-ended needle is retained by the hub and has a proximal end positioned within the port and a distal end extending out from the port. The double-ended needle is movable from a first position where the proximal end is separated from the reservoir to a second position where the proximal end is positioned to pierce into the reservoir. A shield assembly is positioned at the distal end of the end member and may be actuated distally by a biasing member from a retracted position to an advanced position, with the distal end of the needle covered by the shield assembly when in the advanced position.

## Description

### BACKGROUND OF THE INVENTION

### Field of the Invention

The present disclosure relates generally to single-use injection devices and, more specifically, to single-use injection devices with features for automatically disabling repeated use of the device and for shielding a needle of the device post-injection.

### Description of Related Art

Syringes and other injection devices are used in a variety of environments for administering fluids to a patient. When used in controlled healthcare environments and by trained healthcare professionals, such devices provide an effective and cost-efficient solution for the administering of vaccines or other medicaments to a patient. However, it is recognized that issues regarding safety and/or improper use may arise when using injection devices in a less controlled environment. One safety issue that may arise with injection devices such as syringes is the re-use thereof, with re-use of syringes often occurring after inadequate washing and sterilizing thereof, especially in the developing world, and with re-use of a contaminated syringe potentially giving rise to an outbreak or infection. Another safety issue that may arise with syringes is via needle stick injuries that can occur after use of the syringe, with a user of the syringe accidentally sticking themselves with the syringe needle after a fluid has been administering to a patient, so as to potentially subject themselves to a contaminated syringe and a transmissible blood-borne disease. In addition to safety issues, it is recognized that the measuring out and administering of a proper dosage of a medicament to a patient may be subject to error if a user of the syringe is untrained and/or is performing the administering in a chaotic environment.

In order to address some of the aforementioned challenges, many syringes and injection devices have been redesigned to incorporate features thereon that may address issues of safety and/or improper use. As one example, some syringes or injection devices have been designed to incorporate automatic or semi-automatic covering of the needle after use, or modifications that destroy the functionality of a syringe or injection device after one use. These so-called safety syringes/devices are inevitably much more expensive than the standard syringe and, as such, are not a universal solution to address these issues, especially in developing countries that may not be able to afford such syringes.

Besides such safety syringes/devices, other attempts have been made to provide inexpensive, single-use, self-destruct, pre-filled devices, such as may be optimal for use in developing countries. An example of such a device is the "Uniject^{™}" pre-filled injection device made by Becton Dickinson and Company. The Uniject includes a plastic blister or reservoir (containing a medicament) and attached needle, with the device providing a pre-measured dosage of medicament to a patient and including features thereon that prevent the re-use of the device. While the Uniject device thus provides a low-cost solution ideal for use in developing countries and/or for mass vaccination campaigns, existing designs of the device do not provide a mechanism for automatically or semi-automatically covering the needle after use.

Accordingly, a need exists in the art for pre-filled injection devices that effectively disable the device after an initial use and automatically cover the needle after use, while also providing a pre-measured dose of medicament to a patient, with all these features being provided in a device that is affordable and designed for use in uncontrolled environments.

### SUMMARY OF THE INVENTION

Provided herein is a pre-fillable injection device. The injection device includes an end member graspable by a user and having a distal end and a proximal end, the end member comprising a film. The injection device also includes a reservoir defined within the end member and pre-filled with a fluid, with the reservoir is at least partially formed by the film, and with the film being depressible by the user to reduce a volume of the reservoir to force the fluid out therefrom. The injection device further includes a port and a hub provided on the end member at the distal end thereof and configured to provide access to the reservoir, along with a double-ended needle retained by the hub and having a proximal end positioned within the port and a distal end extending out from the port, the double-ended needle movable from a first position where the proximal end is separated from the reservoir to a second position where the proximal end is positioned to pierce into the reservoir, to place the needle in fluid communication with the reservoir. The injection device still further includes a shield assembly positioned at the distal end of the end member and movable distally from a retracted position to an advanced position, with the distal end of the needle extending out distally past the shield assembly when in the retracted position and the distal end of the needle covered by the shield assembly when in the advanced position, as well as a biasing member configured to actuate the shield assembly from the retracted position to the advanced position.

In some embodiments, the pre-fillable injection device includes a removable needle cap, the removable needle cap depressible in a proximal direction to actuate the double-ended needle from the first position to the second position.

In some embodiments, the removable needle cap is removed after actuation of the double-ended needle from the first position to the second position, to enable insertion of the distal end of the needle into a patient.

In some embodiments, the port comprises a safety port including a port body defining a cavity and having an open distal end and a proximal end with an end cover thereat, with the port body having a pair of slots formed therein extending longitudinally along the port body and an opening formed in the port body on a side thereof, at a location between the pair of slots, and a coupling member positioned at a proximal end of the port body and configured to couple the safety port to the end member.

In some embodiments, the shield assembly comprises a needle chamber positionable within the cavity that includes a cylindrical body having an open proximal end, with the cylindrical body having a plurality of slots formed therein extending longitudinally along the cylindrical body and an annular flange formed at a distal end thereof, the annular flange extending radially inward and forming a seat, and a cantilevered flexible tab coupled to a distal end of the cylindrical body, the flexible tab having a button thereon sized to be received within the opening of the port body. The shield assembly also comprises and a pair of side members configured to movably couple the needle chamber to the safety port, each of the pair of side member comprising pins and locking tabs that engage with the pair of slots of the port body and the plurality of slots of the cylindrical body, to enable a longitudinal movement of the pair of side members and the needle chamber relative to the safety port. The biasing member is positioned between the safety port and the needle chamber and retained by the end cover and the seat, the biasing configured to translate the needle chamber distally out from the safety port, thereby transitioning the shield assembly from the retracted position to the advanced position.

In some embodiments, when the flexible tab is depressed inwardly, the button is disengaged from the opening to unlock the needle chamber from the safety port, thereby releasing the biasing member and enabling the distal movement of the needle chamber distally out from the safety port.

In some embodiments, engagement of the pins with the pair of slots of the port body and the plurality of slots of the cylindrical body limit the distal movement of the needle chamber distally out from the safety port, to prevent over-sliding.

In some embodiments, upon transitioning of the shield assembly to the advanced position, the locking tabs lock the needle chamber in place and prevent a transition back to the retracted position.

In some embodiments, the removable needle cap is coupled to the distal end of the cylindrical body of the needle chamber.

In some embodiments, the pre-fillable injection device includes a guide member coupled to the port, with the guide member including an elongated frame attached to the port and extending across a width of the end member, the elongated frame including a first through-hole positioned adjacent a first edge of the elongated frame, a second through-hole positioned adjacent a second edge of the elongated frame, and a third through-hole centered on the elongated frame and aligned with the port, with a seat formed about the third through-hole on a distal-facing side of the elongated frame. The guide member also includes a pair of release tabs formed at opposing sides of the elongated frame and adjacent to the first and second through-holes.

In some embodiments, the shield assembly comprises a U-shaped slide member including a cross-bracket having a first end and a second end, a first sliding guide positioned at the first end of the cross-bracket and extending proximally from the cross-bracket, the first sliding guide configured to slide through the first through-hole, a second sliding guide positioned at the second end of the cross-bracket and extending proximally from the cross-bracket, the second sliding guide configured to slide through the second through-hole, and a receptacle centered on the cross-bracket; and a safety chamber positioned within the receptacle, the safety chamber having an open proximal end and an open distal end. The biasing member is positioned between the guide member and the U-shaped slide member and retained by the seat and the receptacle, the biasing member configured to translate the U-shaped slide member distally away from the guide member, thereby transitioning the shield assembly from the retracted position to the advanced position.

In some embodiments, each of the first sliding guide and the second sliding guide comprises a pair of distal support tabs and a pair of proximal support tabs, wherein each of the pair of distal support tabs is engageable with a notch formed a respective one of the first through-hole and the second through-hole, and wherein the pair of proximal support tabs are engageable with proximal- and distal-facing sides of the elongated frame.

In some embodiments, with the shield assembly in the retracted position, one of the pair of distal support tabs on each of the first and second sliding guides is engaged with the notch in the respective one of the first through-hole and the second through-hole, to lock the U-shaped slide member in the retracted position.

In some embodiments, when the pair of release tabs are depressed inwardly, the one of the pair of distal support tabs on each of the first and second sliding guides button is disengaged from the notch to unlock the U-shaped slide member from the guide member, thereby releasing the biasing member and enabling the distal movement of the U-shaped slide member away from the guide member.

In some embodiments, with the shield assembly in the advanced position, the pair of proximal support tabs engage the proximal- and distal-facing sides of the elongated frame, to lock the U-shaped slide member in place and prevent a transition back to the retracted position.

In some embodiments, with the shield assembly in the advanced position, the distal end of the double-ended needle is positioned within the safety chamber.

In some embodiments, the shield assembly further comprises a flexible hook coupled to an inner surface of the safety chamber, the flexible hook positioned alongside the needle and deflected thereby when the shield assembly is in the retracted position and positioned in an un-deflected position that covers the distal end of the needle when the shield assembly is in the advanced position.

In some embodiments, the hub comprises a pair of slots formed therein extending longitudinally along the hub.

In some embodiments, the shield assembly comprises a needle guard coupled to the hub, with the needle guard including a cylindrical cover portion having a closed distal end with a needle hole formed therein through which the needle may pass, and a pair of fingers extending out proximally from the cylindrical cover portion and configured to engage and slide within the pair of slots, wherein the needle guard is rotatable between a locked position that prevents proximal movement of the needle guard relative to the hub and an unlocked position that allows proximal movement of the needle guard relative to the hub, with the pair of fingers misaligned with the pair of slots when in the locked position and aligned with the pair of slots when in the unlocked position.

In some embodiments, each of the port and the needle guard includes markings thereon to identify a rotational position of the needle guard as either in the locked position or the unlocked position.

In some embodiments, the biasing member is positioned between a distal end of the hub and the closed distal end of the needle guard, with the biasing member configured to apply a biasing force to the needle guard that biases the needle guard to the advanced position.

In some embodiments, when the needle guard is in the unlocked position and a proximally directed pushing force is applied to the needle guard that is greater than the biasing force, the needle guard is moved from the advanced position to the retracted position, with the pair of fingers sliding proximally into the pair of slots.

In some embodiments, when the needle guard is in the locked position, the needle guard is depressible in a proximal direction to actuate the double-ended needle from the first position to the second position.

In some embodiments, the biasing member comprises a spring configured to transition from a compressed state to an extended state, the spring transitioning from the compressed state to the extended state to move the shield assembly from the retracted position to the advanced position.

### BRIEF DESCRIPTION OF THE DRAWINGS

FIG. 1 is a perspective view of an injection device, according to a non-limiting embodiment described herein;
FIG. 2 is an exploded view of the injection device of FIG. 1;
FIG. 3 is a perspective view of a shield assembly included in the injection device of FIG. 1;
FIG. 4 is a side cross-sectional view of the shield assembly of FIG. 3;
FIG. 5 is a side view of the injection device of FIG. 1, with the injection device in an initial condition;
FIG. 6 is a side cross-sectional view of the injection device of FIG. 1, with the injection device in an activated condition and with the shield assembly in a retracted position;
FIG. 7 is a side cross-sectional view of the injection device of FIG. 1, with a needle cap removed, prior to an initial use;
FIG. 8 is a side cross-sectional view of the injection device of FIG. 1, with the shield assembly in an advanced position, subsequent to an initial use;
FIG. 9 is a perspective view of an injection device, according to another non-limiting embodiment described herein;
FIG. 10 is an exploded view of the injection device of FIG. 9;
FIG. 11 is a side view of the injection device of FIG. 9, with the injection device in an initial condition;
FIG. 12 is a side cross-sectional view of the injection device of FIG. 9, with the injection device in an activated condition and with a shield assembly thereof in a retracted position;
FIG. 13 is a side view of the injection device of FIG. 9, with a needle cap removed, prior to an initial use;
FIG. 14A is a side view of the injection device of FIG. 9, with the shield assembly in an advanced position, subsequent to an initial use;
FIG. 14B is a side cross-sectional view of the injection device of FIG. 9, with the shield assembly in an advanced position, subsequent to an initial use;
FIG. 15 is a perspective view of an injection device, according to another non-limiting embodiment described herein;
FIG. 16 is an exploded view of the injection device of FIG. 15;
FIG. 17 is a side cross-sectional view of the injection device of FIG. 15, with the injection device in an initial condition;
FIG. 18 is a side cross-sectional view of the injection device of FIG. 15, with the injection device in an activated condition and with a shield assembly thereof in a locked and advanced position, prior to an initial use;
FIG. 19 is a side cross-sectional view of the injection device of FIG. 15, with the injection device in an activated condition and with a shield assembly thereof in an unlocked and advanced position, prior to an initial use;
FIG. 20 is a side cross-sectional view of the injection device of FIG. 15, with the shield assembly pushed proximally to a retraced position, during an initial use;
FIG. 21 is a side cross-sectional view of the injection device of FIG. 15, with the shield assembly pushed back distally to the advanced position, subsequent to an initial use; and
FIG. 22 is a side cross-sectional view of the injection device of FIG. 15, with the shield assembly in the locked and advanced position, subsequent to an initial use.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, equivalents, variations, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

As used in this specification, the words "proximal" and "distal" refer to the direction closer to and away from, respectively, a user who would place the device into contact with a patient. Thus, for example, the end of a device first touching the body of the patient would be the distal end, while the opposite end of the device being manipulated by the user would be the proximal end of the device.

The terms "first", "second", and the like are not intended to refer to any particular order or chronology, but refer to different conditions, properties, or elements.

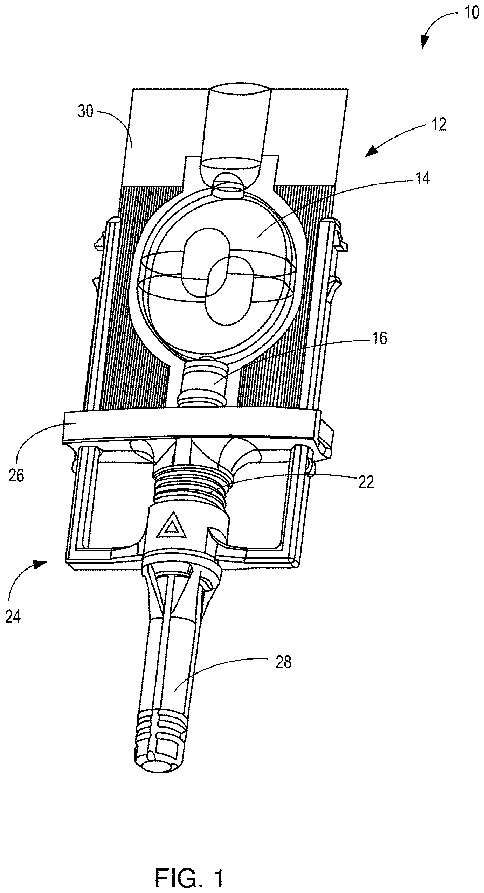

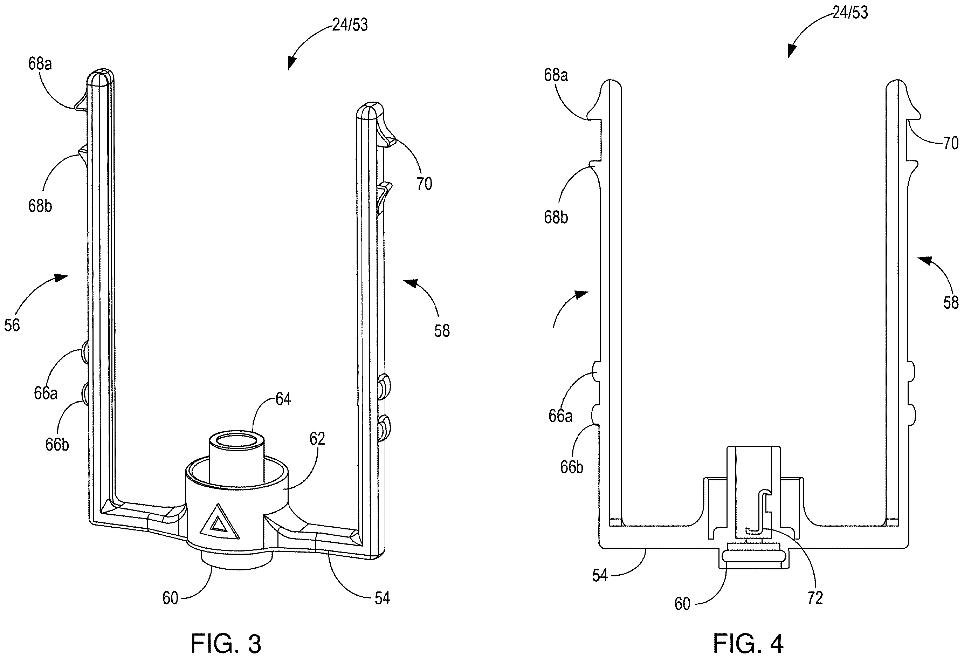

Referring to FIGS. 1-8, shown is a non-limiting embodiment of a pre-filled or pre-fillable medical liquid injection device 10 for injecting a medicament into a patient. The injection device 10 generally includes an end member 12 that defines a reservoir 14, an outlet or port 16 in fluid communication with the reservoir 14, a hub 18, a double-ended needle cannula 20 (hereafter "needle 20"), a biasing member 22, a shield assembly 24, a guide member 26, and a removable needle cap 28.

In accordance with aspects of the disclosure, the end member 12 is configured to include a peripheral region 30 that surrounds the reservoir 14. Portions of or an entirety of the end member 12 may have a laminate construction and be formed from a number of films that are sealed/joined together. In some embodiments, the end member 12 includes a laminate comprised of an inner layer film of low density polyethylene and an outer layer film of a cyclic olefin or cyclic olefin copolymer, with the inner layer film and the outer layer film preferably coextruded and then thermoformed. The peripheral region 30 may be generally flat and shaped to enable a grasping thereof by a user, while the reservoir 14 is surrounded by the peripheral region 30 and is configured as a bulb-shaped reservoir. In at least the region of the bulb-shaped reservoir 14, the laminate film(s) forming end member 12 are formed of a deformable material, such that each of opposing sides of the bulb-shaped reservoir 14 may be inwardly depressed to shrink a volume of the reservoir 14 during use of the injection device 10. Upon depression of the bulb-shaped reservoir 14, a single dose of a medical liquid contained therein may be forced out of the reservoir 14 and through the needle 20 for injection into a patient, as described in further detail below. During manufacturing of the injection device 10, the reservoir 14 may be filled with a filler tube (not shown) in fluid communication with the reservoir 14 and then sealed.

The port 16 of injection device 10 is provided at a distal end 32 of the end member 12 and is configured as a tubular port that includes therein a pierceable membrane 34 that seals the reservoir 14. The hub 18 is positioned within the port 16 and seated therein, with the hub 18 configured to retain the needle 20 (i.e., the needle 20 is secured within a hole formed through hub 18). The hub 18 may be seated within port 16 so as to movable therein if a sufficient force is applied against the hub 18. That is, the hub 18 may be moved proximally back and further into the port 16 when a force is applied thereto.

The needle 20 includes a proximal sharp end 36 that is positioned within an internal volume of the port 16 and an opposed distal sharp end 38 that is initially received within (and covered by) the needle cap 28. The distal end 38 is used for aspiration or injection of the medicament contained within reservoir 14 into a patient. During use of the injection device 10, the proximal end 36 of the needle 20 is received within the port 16 but is separated from the pierceable membrane 34. The proximal end 36 of the needle 20 may be driven through the pierceable membrane 34 responsive to a user pushing the needle cap 28, with the hub 18 and needle 20 thus being forced back proximally further into the port 16, until the proximal end 36 of the needle 20 pierces through the membrane 34, so as to place the needle 20 in fluid communication with the reservoir 14.

According to aspects of the disclosure, and as indicated above, injection device 10 includes a biasing member 22, a shield assembly 24, and a guide member 26 that, collectively, provide for the covering of the distal end 38 of the needle 20 subsequent to an injection being performed. The shield assembly 24 is positioned at the distal end 32 of the end member 12 and, via a sliding interaction with the guide member 26, is movable distally from a retracted position to an advanced position. When the shield assembly 24 is in the retracted position, the distal end 38 of the needle 20 extends out distally past the shield assembly 24. When the shield assembly 24 is actuated from the retracted position to the advanced position by the biasing member 22 (e.g., a spring), the distal end 38 of the needle 20 is covered by the shield assembly 24.

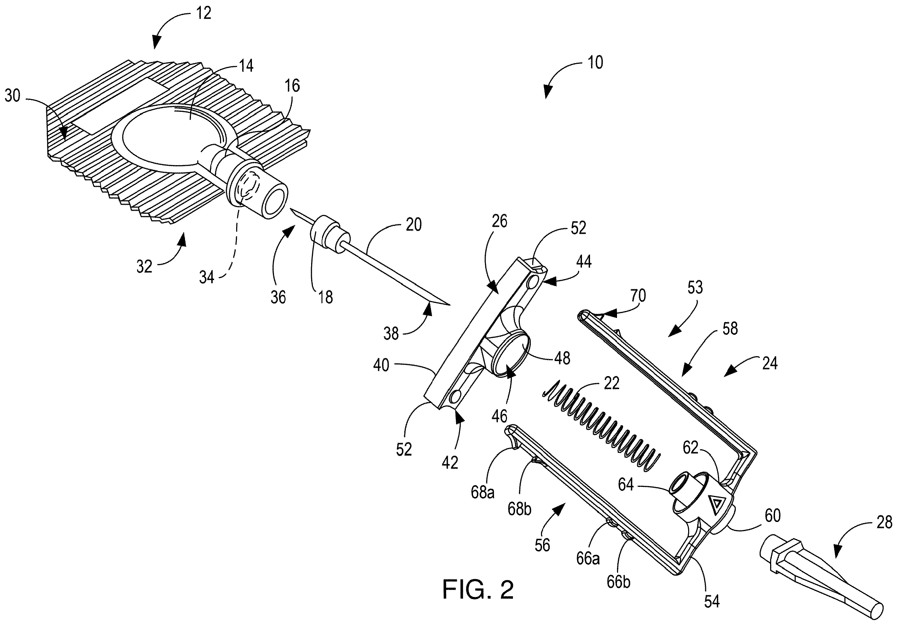

The guide member 26 includes an elongated frame 40 that is coupled to the port 16 and positioned adjacent the distal end 32 of the end member 12. The elongated frame 40 extends across a width of the end member 12 and includes a series of through holes formed therein, including a first through-hole 42 positioned adjacent a first edge of the elongated frame 40, a second through-hole 44 positioned adjacent a second edge of the elongated frame 40, and a third through-hole 46 centered on the elongated frame 40 and aligned to receive the port 16 therethrough, with a seat 48 (FIGS. 2 and 6-8) formed about the third through-hole 46 on a distal-facing side of the elongated frame 40. Each of the first and second through-holes 42, 44 includes a notch 50 (or opening) formed on an inner surface thereof that extends radially outward. The guide member 26 also includes a pair of release tabs 52 formed at opposing sides of the elongated frame 40 and adjacent to the first and second through-holes 42, 44, with the release tabs 52 aligned with the notches/openings 50 formed in the through-holes 42, 44. The release tabs 52 are depressible inward to selectively enable or inhibit movement of the shield assembly 24, as explained in further detail below.

According to aspects of the disclosure, the shield assembly 24 is constructed as a U-shaped slide member 53 that includes a cross-bracket 54, a first sliding guide 56, and a second sliding guide 58. The first sliding guide 56 is positioned at a first end of the cross-bracket 54 and extends back proximally therefrom, with the first sliding guide 56 configured to slide through the first through-hole 42 of the guide member 26. The second sliding guide 58 is positioned at a second end of the cross-bracket 54 and extends back proximally therefrom, with the second sliding guide 58 configured to slide through the second through-hole 44 of the guide member 26.

According to embodiments, a needle cap coupler 60, a receptacle 62, and a safety chamber 64 are all provided on the cross-bracket 54, at a central location thereon. The needle cap coupler 60 is formed on a distal facing surface of the cross-bracket 54 and forms an engagement feature with which the needle cap 28 may be connected. The receptacle 62 and the safety chamber 64 are formed on a proximal facing surface of the cross-bracket 54 and extend proximally therefrom, with the receptacle 62 and the safety chamber 64 arranged in a concentric manner such that the safety chamber 64 is positioned within the receptacle 62. The safety chamber 64 is a tube-shaped member that includes an open proximal end and a closed distal end, while the receptacle 62 has an open proximal end and a closed distal end. The receptacle 62 is configured to receive a distal end of the biasing member 22 therein, while a proximal end of the biasing member 22 is retained within the seat 48 formed in/on the elongated frame 40. The biasing member 22 is thus positioned between the guide member 26 and the U-shaped slide member 53, to provide for translation of the U-shaped slide member 53 distally away from the guide member 26 (i.e., from the retracted position to the advanced position) when the biasing member 22 transitions from a compressed state to an extended state.

According to embodiments, each of the first sliding guide 56 and the second sliding guide 58 comprises a pair of distal support tabs 66a, 66b formed near a distal end thereof and a pair of proximal support tabs 68a, 68b formed near a proximal end thereof. The support tabs forming the pair of distal support tabs 66a, 66b may be configured as rounded elastic bumps and may be spaced apart from each other along a length of each sliding guide 56, 58, with the bumps engageable with the notches/openings 50 formed in the first and second through-holes 42, 44. The support tabs forming the pair of proximal support tabs 68a, 68b may be configured as ramps that include a flat end surface 70, with the flat end surfaces 70 of the tabs 68a, 68b facing each other. The proximal support tabs 68a, 68b may be spaced apart from each other along a length of each sliding guide 56, 58.

In some embodiments, the shield assembly 24 may further include a flexible hook 72 that is coupled to an inner surface of a cylindrical wall that forms the safety chamber 64. The flexible hook 72 comprises a biased member that may move between a deflected position and an un-deflected position, depending on whether the shield assembly 24 is positioned in the retracted position or the advanced position. When the shield assembly 24 is positioned in the retracted position, the flexible hook 72 will be positioned alongside the needle 20 and deflected thereby. When the shield assembly 24 is in the advanced position, the flexible hook 72 will be positioned distal from the distal end 38 of the needle 20, and is thus free to move to its un-deflected position, with the flexible hook 72 thus positioned to cover the distal end 38 of the needle 20.

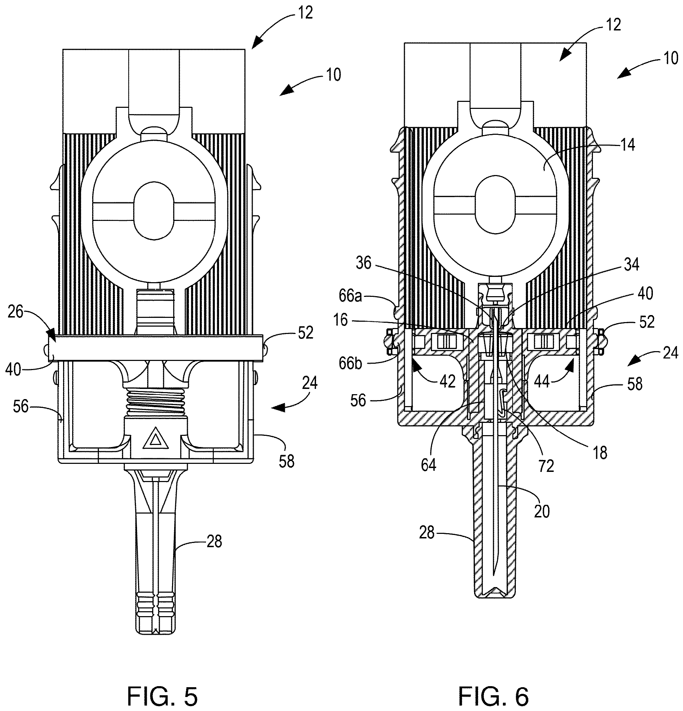

Referring now to FIGS. 5-8, operation of the injection device 10 is described in more detail. From an initial configuration of the injection device 10, as shown in FIG. 5, a user will first push the needle cap 28 proximally, in order to place the needle 20 in fluid communication with the reservoir 14, as shown in FIG. 6. When the needle cap 28 is pushed back proximally, the shield assembly 24 is also forced to move back proximally - with the first and second sliding guides 56, 58 sliding back within the through-holes 42, 44 of guide member 26. The proximal movement of the first and second sliding guides 56, 58 causes the proximal-most tab 66a of the pair of distal support tabs 66a, 66b (on each sliding guide) to be forced out of engagement with its respective notch/opening 50 in the through-holes 42, 44, with the first and second sliding guides 56, 58 continuing to move proximally back until the distal-most tab 66b of the pair of distal support tabs 66a, 66b (on each sliding guide) is brought into engagement with its respective notch/opening 50 in the through-holes 42, 44. This proximal movement of the shield assembly 24 is transferred to the hub 18 that retains the needle 20, with a proximal end of the safety chamber 64 abutting the hub 18 and forcing it in the proximal direction. The proximal movement of the hub 18 causes the proximal end 36 of the needle 20 to be driven through the pierceable membrane 34, so as to place the needle 20 in fluid communication with the reservoir 14.

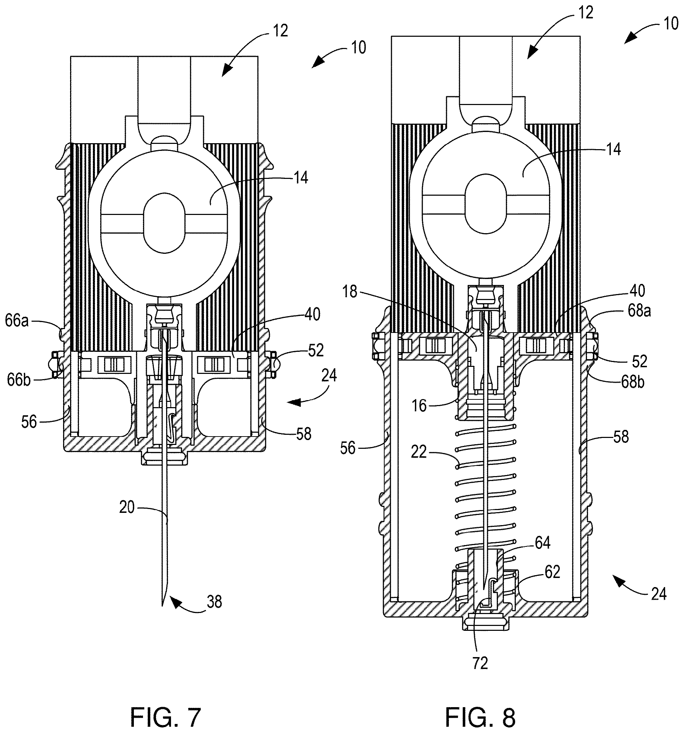

As shown in FIG. 7, upon placing of the needle 20 in fluid communication with the reservoir 14, the needle cap 28 may be removed (i.e., disengaged from cap coupler 60), in order to prepare for injection of the medicament into a patient. Upon insertion of the distal end 38 of the needle 20 into the patient, the bulb-shaped reservoir 14 may be depressed (on both sides thereof) by a user, to force the medicament out from the reservoir 14 and through the needle 20, for administering to the patient.

With the distal end 38 of the needle 20 still inserted within the patient (or immediately after being removed from the patient), the user may then press and hold the pair of release tabs 52 formed at opposing sides of the elongated frame 40, in order to unlock the shield assembly 24 from the guide member 26. With the release tabs 52 depressed, the elastic bumps 66a, 66b on the first and second sliding arms 56, 58 are disengaged from the guide member 26, allowing for movement of the shield assembly 24 (i.e., U-shaped slide member 53) from its retracted position to its advanced position, via transitioning of the biasing member 22 from its compressed state to its extended state. As the injection device 10 (and the needle 20 thereof) is withdrawn proximally away from the patient, the biasing member 22 forces the shield assembly 24 distally toward its advanced position. The shield assembly 24 thus moves distally toward its advanced position, until the pair of proximal support tabs 68a, 68b engage the proximal- and distal-facing sides of the elongated frame 40, as shown in FIG. 8. That is, the shield assembly 24 will move distally toward its advanced position until the elongated frame 40 is moved between the pair of distal support tabs 66a, 66b - with the flat end surface 70 of each of the tabs engaging the proximal and distal-facing surfaced of the elongated frame 40 to lock the shield assembly 24 (i.e., the U-shaped slide member) in place and prevent a transition back to the retracted position. With the shield assembly 24 in the advanced position, the distal end 38 of the needle 20 is positioned within the safety chamber 64 and the flexible hook 72 is positioned distal from the distal end 38 of needle 20, in its un-deflected position, so as to cover the distal end 38 of the needle 20.

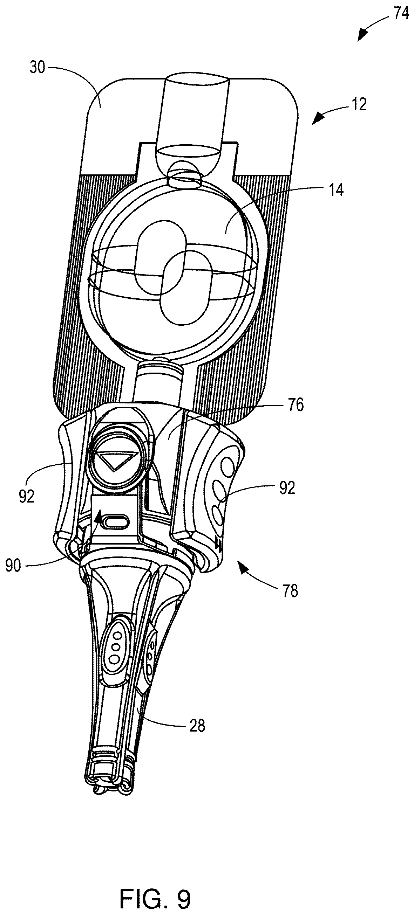

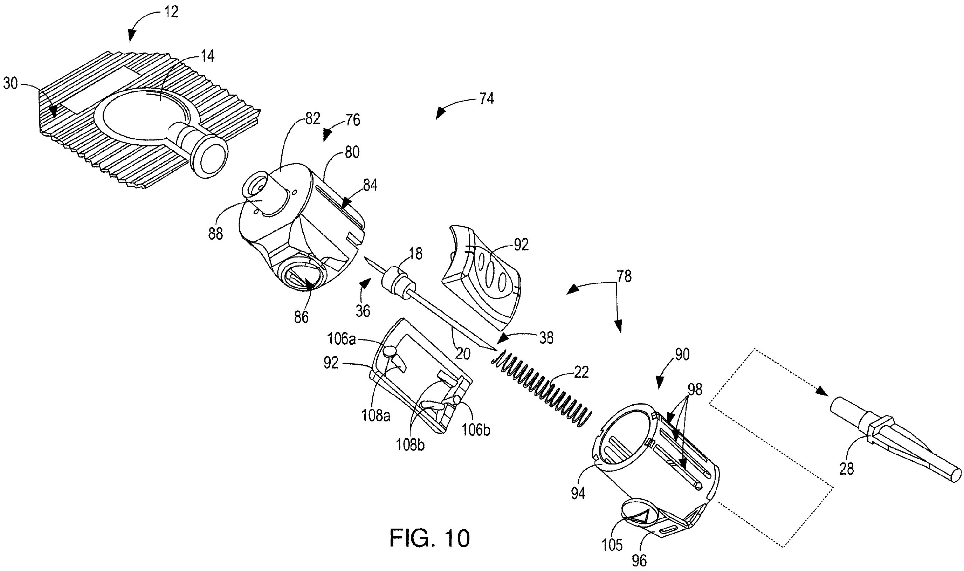

Referring now to FIGS. 9-14 an embodiment of an injection device 74 is illustrated, in accordance with another aspect of the disclosure. The injection device 74 generally includes an end member 12 that defines a reservoir 14, a port 76 in fluid communication with the reservoir 14, a hub 18, a double-ended needle 20, a biasing member 22, a shield assembly 78, and a removable needle cap 28.

As previously described regarding the injection device 10 of FIGS. 1-8, the end member 12 is configured to include a peripheral region 30 that surrounds the reservoir 14. Portions of or an entirety of the end member 12 may have a laminate construction and be formed from a number of films that are sealed/joined together. The peripheral region 30 may be generally flat and shaped to enable a grasping thereof by a user, while the reservoir 14 is surrounded by the peripheral region 30 and is configured as a bulb-shaped reservoir - with each of opposing sides of the bulb-shaped reservoir 14 configured to be inwardly depressed to shrink a volume of the reservoir 14 during use of the injection device 74 and force a single dose of a medical liquid contained therein out of the reservoir 14 and through the needle 20 for injection into a patient.

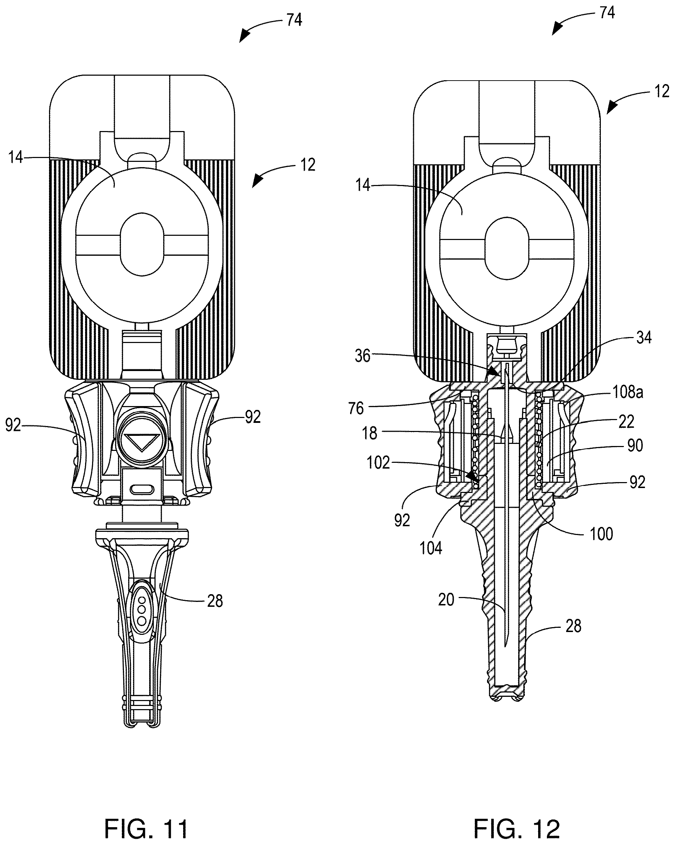

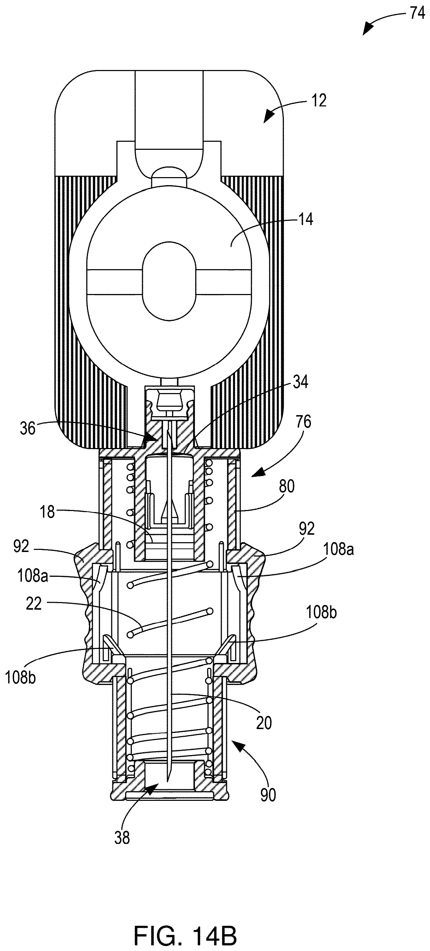

The port of injection device 74 is provided at a distal end 32 of the end member 12 and includes therein a pierceable membrane 34 (FIGS. 12 and 14B) that seals the reservoir 14. According to aspects of the disclosure, the port 76 is configured as a safety port (hereafter "safety port 76") that includes a port body 80 defining a cavity with an open distal end and a proximal end with an end cover 82 thereat. The port body 80 also includes a pair of slots 84 formed therein extending longitudinally therealong, as well as an opening 86 formed in the port body 80 on a side thereof, at a location between the pair of slots 84. A coupling member 88 is positioned at a proximal end of the port body 80 and configured to couple the safety port 76 to the end member 12, with the pierceable membrane 34 that seals the reservoir 14 being positioned in the coupling member 88.

The hub 18 is positioned within the safety port 76 and seated therein, with the hub 18 configured to retain the needle 20 (i.e., the needle 20 is secured within a hole formed through hub 18). The hub 18 may be seated within safety port 76 so as to be movable therein if a sufficient force is applied against the hub 18. In some embodiments, the hub 18 may be moved proximally back further into the safety port 76 to a position where the needle 20 is brought into fluid communication with the reservoir 14 (i.e., where the needle 20 pierces the membrane 34 sealing the reservoir 14).

The needle 20 includes a proximal sharp end 36 that is positioned within an internal volume of the safety port 76 and an opposed distal sharp end 38 that is initially received within (and covered by) the needle cap 28. The distal sharp end 38 is used for aspiration or injection of the medicament contained within reservoir 14 into a patient. During use of the injection device 74, the proximal end 36 of the needle 20 is received within the safety port 76 but is separated from the pierceable membrane 34. The proximal end 36 of the needle 20 may be driven through the pierceable membrane 34 responsive to a user pushing the needle cap 28 proximally, with the hub 18 and needle 20 thus being forced back proximally further into the safety port 76 until the proximal end 36 of the needle 20 pierces through the membrane 34, so as to place the needle 20 in fluid communication with the reservoir 14.

According to aspects of the disclosure, and as indicated above, injection device 74 includes a biasing member 22 and a shield assembly 78 that, collectively, provide for the covering of the distal end 38 of the needle 20 subsequent to an injection being performed. The shield assembly 78 is positioned at the distal end 32 of the end member 12 and, via a sliding interaction with the safety port 76, is movable distally from a retracted position to an advanced position. When the shield assembly 78 is in the retracted position, the distal end 38 of the needle 20 extends out distally past the shield assembly 78. When the shield assembly 78 is actuated from the retracted position to the advanced position by the biasing member 22 (e.g., a spring), the distal end 38 of the needle 20 is covered by the shield assembly 78.

According to aspects of the disclosure, the shield assembly 78 includes a needle chamber 90 and a pair of side members 92. The needle chamber 90 is positionable within the cavity of the safety port 76 and includes a cylindrical body 94 and a cantilevered flexible tab 96 coupled to a distal end of the cylindrical body 94. The cylindrical body 94 has an open proximal end and includes a plurality of slots 98 formed therein extending longitudinally therealong, as well as an annular flange 100 formed at a distal end thereof. The annular flange 100 extends radially inward and forms a proximally-facing seat 102 in/on the needle chamber 90. In some embodiments, the distal end of the cylindrical body 94 also includes a distally-facing needle cap coupler 104 that provides an engagement feature with which the needle cap 28 may be coupled. The cantilevered flexible tab 96 is coupled to the distal end of the cylindrical body 94 (at a distal end of the flexible tab 96) and includes a release button 105 thereon (at a proximal end of the flexible tab 96) that is sized to be received within the opening 86 of the port body 80. The cantilevered flexible tab 96 may be pressed/flexed radially inward via application of a pushing force to the release button 105, to provide for disengagement of the button 105 from the opening 86 of port body 80, thereby unlocking the needle chamber from the safety port 76.

The pair of side members 92 of the shield assembly 78 are configured to movably couple the needle chamber 90 to the safety port 76. Each of the pair of side members 92 includes pins 106 formed on an inward-facing surface thereof that engage with the pair of slots 84 of the port body 80 and the plurality of slots 98 of the cylindrical body 94, to enable a longitudinal movement of the pair of side members 92 and the needle chamber 90 relative to the safety port 76. Each of the pair of side members 92 also includes locking tabs 108 formed on an inward-facing surface thereof that engage with the pair of slots 84 of the port body 80 and the plurality of slots 98 of the cylindrical body 94, to limit the distal movement of the needle chamber 90 distally out from the safety port 76, to prevent over-sliding and disengagement. In some embodiments, each of the side members 92 includes a top pin 106a and a bottom pin 106b spaced apart in a longitudinal direction, along with a top locking tab 108a (aligned with the top pin) and a pair of bottom locking tabs 108b spaced apart in a longitudinal direction from the top locking tab 108a and spaced apart from one another in a horizontal direction.

The biasing member 22 is positioned relative to the safety port 76 and the needle chamber 90 so as to be retained therebetween and provide for translation of the needle chamber 90 (and side members 92) distally away from the safety port 76 (i.e., translate the shield assembly 78 from the retracted position to the advanced position) when the biasing member 22 transitions from a compressed state to an extended state. More specifically, the biasing member 22 is retained between/by the end cover 82 of safety port 76 and the seat 102 of needle chamber 90.

Referring now to FIGS. 11-14, operation of the injection device 74 is described in more detail. From an initial configuration of the injection device 74, as shown in FIG. 11, a user will first push the needle cap 28 proximally, in order to place the needle 20 in fluid communication with the reservoir 14, as shown in FIG. 12. When the needle cap 28 is pushed back proximally, the shield assembly 78 is also forced to move back proximally - with the needle chamber 90 being pushed back within the safety port 76. The proximal movement of the needle chamber 90 is transferred to the hub 18 that retains the needle 20, with a proximal end of the needle chamber 90 abutting the hub 18 and forcing it in the proximal direction. The proximal movement of the hub 18 causes the proximal end 36 of the needle 20 to be driven through the pierceable membrane 34, so as to place the needle 20 in fluid communication with the reservoir 14.

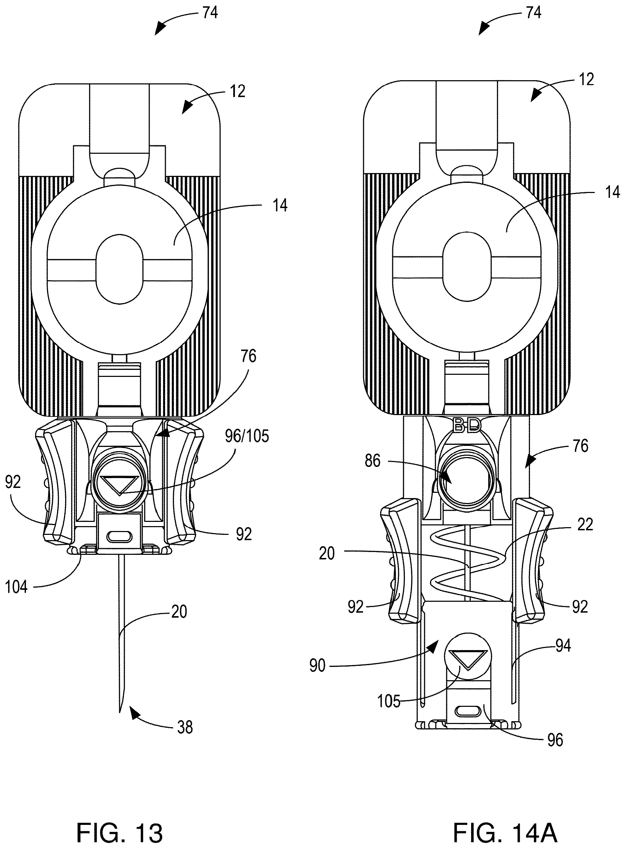

As shown in FIG. 13, upon placing of the needle 20 in fluid communication with the reservoir 14, the needle cap 28 may be removed (i.e., disengaged from cap coupler 104), in order to prepare for injection of the medicament into a patient. Upon insertion of the distal end 38 of the needle 20 into the patient, the bulb-shaped reservoir 14 may be depressed (on both sides thereof) by a user, to force the medicament out from the reservoir 14 and through the needle 20, for administering to the patient.

With the distal end 38 of the needle 20 still inserted within the patient, the user may then press and hold the release button 105 formed on the cantilevered flexible tab 96 of the needle chamber 90, in order to unlock the needle chamber 90 from the safety port 76. With the release button 105 depressed, the button 105 is disengaged from the opening 86 of the safety port 76, allowing for movement of the shield assembly 78 (i.e., of the needle chamber 90 and the side members 92) from its retracted position to its advanced position, via transitioning of the biasing member 22 from its compressed state to its extended state. As the injection device 74 (and the needle 20 thereof) is withdrawn proximally away from the patient, the biasing member 22 forces the shield assembly 78 distally toward its advanced position. The shield assembly 78 thus moves distally toward its advanced position - via sliding of the pins 106 of side members 92 within the slots 98 of the port body 80 and the slots 84 of the cylindrical body 94 - until the locking tabs 108 of side members 92 reach a distal end of the slots 98, 84 in the port body 80 and the cylindrical body 94, as shown in FIGS. 14A and 14B. That is, the shield assembly 78 will move distally toward its advanced position until the locking tabs 108 restrict further movement thereof and lock the shield assembly 78 (i.e., the needle chamber 90 and side members 92) in place to prevent a transition back to the retracted position. With the shield assembly 78 in the advanced position, the distal end 38 of the double-ended needle 20 is positioned within the needle chamber 90, so as to cover the distal end 38 of the needle 20.

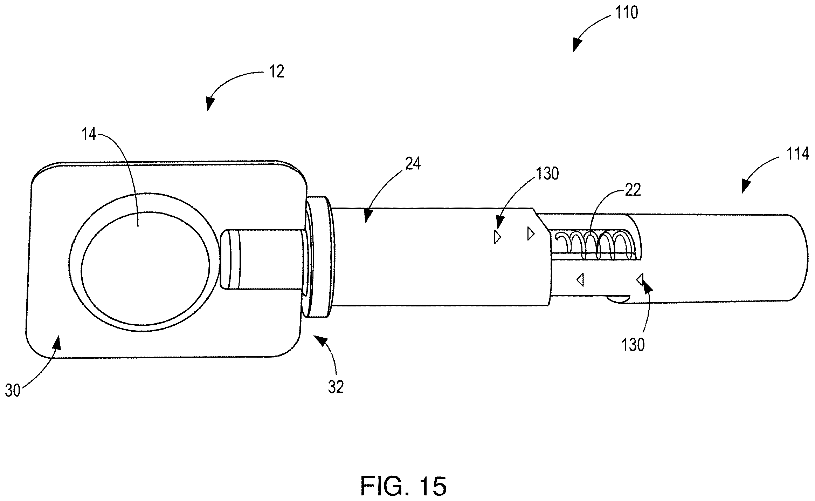

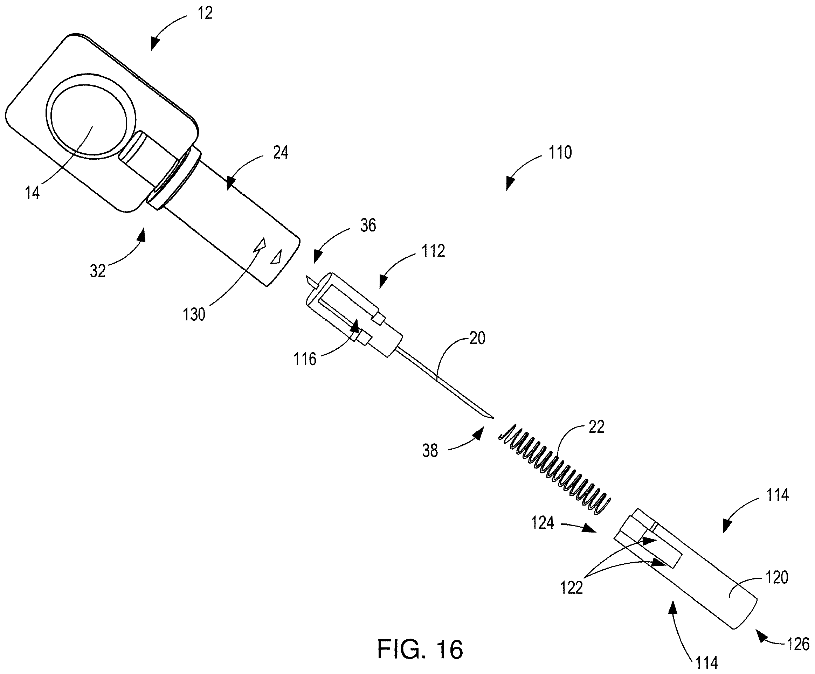

Referring now to FIGS. 15-22 an embodiment of an injection device 110 is illustrated, in accordance with another aspect of the disclosure. The injection device 110 generally includes an end member 12 that defines a reservoir 14, a port 24 in fluid communication with the reservoir 14, a hub 112, a double-ended needle 20, a biasing member 22, and a shield assembly 114.

As previously described regarding the injection devices 10, 74 of FIGS. 1-8 and FIGS. 9-14, the end member 12 is configured to include a peripheral region 30 that surrounds the reservoir 14. Portions of or an entirety of the end member 12 may have a laminate construction and be formed from a number of films that are sealed/joined together. The peripheral region 30 may be generally flat and shaped to enable a grasping thereof by a user, while the reservoir 14 is surrounded by the peripheral region 30 and is configured as a bulb-shaped reservoir - with each of opposing sides of the bulb-shaped reservoir 14 configured to be inwardly depressed to shrink a volume of the reservoir 14 during use of the injection device 110 and force a single dose of a medical liquid contained therein out of the reservoir 14 and through the needle 20 for injection into a patient.

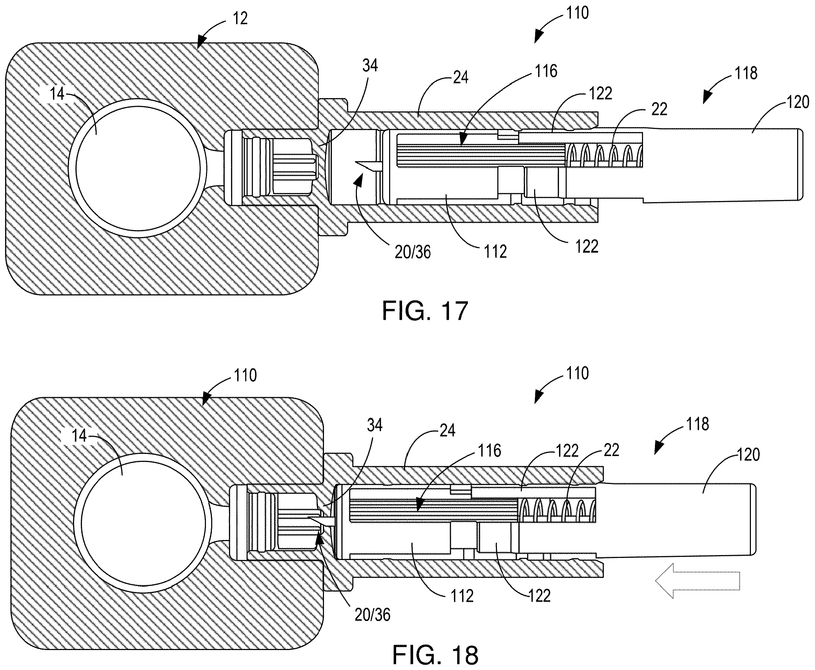

The port 24 of injection device 110 is provided at a distal end 32 of the end member 12 and is configured as a tubular port that includes therein a pierceable membrane 34 (FIGS. 17-22) that seals the reservoir 14. The hub 112 is positioned within the port 24 and seated therein, with the hub 112 configured to retain the needle 20 (i.e., the needle 20 is secured within a hole formed through hub 112). The hub 112 may be seated within port 24 so as to be movable therein if a sufficient force is applied against the hub 112. In some embodiments, the hub 112 may be moved proximally back further into the port 24 to a position where the needle 20 is brought into fluid communication with the reservoir 14 (i.e., where the needle 20 pierces the membrane 34 sealing the reservoir 14).

According to aspects of the disclosure, the hub 112 is configured to include a pair of slots 116 formed therein that extend longitudinally along the hub 112. The pair of slots 116 may be on opposing sides/surfaces of the hub 112 and extend a majority of the length of the hub 112. The slots 116 may be open at the distal end of the hub 112 to provide for engagement with the shield assembly 114, as explained in further detail below, while the slots 116 may be closed at the proximal end of the hub 112.

The needle 20 includes a proximal sharp end 36 that is positioned within an internal volume of the port 24 and an opposed distal sharp end 38 that is initially received within (and covered by) the shield assembly 114. The distal sharp end 38 is used for aspiration or injection of the medicament contained within reservoir 14 into a patient. During use of the injection device 110, the proximal end 36 of the needle 20 is received within the port 24 but is separated from the pierceable membrane 34. The proximal end 36 of the needle 20 may be driven through the pierceable membrane 34 responsive to a user pushing the shield assembly 114 proximally against the hub 112, with the hub 112 and needle 20 thus being forced back proximally further into the port 24 until the proximal end 36 of the needle 20 pierces through the membrane 34, so as to place the needle 20 in fluid communication with the reservoir 14.

According to aspects of the disclosure, and as indicated above, injection device 110 includes a biasing member 22 and a shield assembly 114 that, collectively, provide for the covering of the distal end 38 of the needle 20 subsequent to an injection being performed. The shield assembly 114 is positioned at the distal end 32 of the end member 12 and, via a sliding interaction with the hub 112 and port 24, is movable between an advanced position and a retracted position. When the shield assembly 114 is in the retracted position, the distal end 38 of the needle 20 extends out distally past the shield assembly 114. When the shield assembly 114 is actuated from the retracted position to the advanced position by the biasing member 22 (e.g., a spring), the distal end 38 of the needle 20 is covered by the shield assembly 114.

According to aspects of the disclosure, the shield assembly 114 is provided as a needle guard 118 that is movably coupled to the hub 112. The needle guard 118 may be characterized as generally including a cylindrical cover portion 120 and a pair of fingers 122. The cylindrical cover portion 120 includes an open proximal end 124 and a closed distal end 126, with a needle hole 128 formed in the closed distal end 126 through which the needle 20 may pass. The pair of fingers 122 extend out proximally from the cylindrical cover portion 120 and are configured to engage and slide within the pair of slots 116 formed in hub 112. The biasing member 22 is positioned between a distal end of the hub 112 and the closed distal end 126 of the needle guard 118, with the biasing member 22 configured to apply a biasing force to the needle guard 118 that biases the needle guard 118 to the advanced position.

According to aspects of the disclosure, the needle guard 118 is rotatable relative to the hub 112 and the port 24 in a clockwise and/or counterclockwise manner, such that the needle guard 118 may be rotated between a locked position that prevents proximal movement of the needle guard 118 into engagement with the hub 112 and an unlocked position that allows proximal movement of the needle guard 118 into engagement with the hub 112. That is, when the needle guard 118 is in the locked position, the pair of fingers 122 are misaligned with the pair of slots 116 in the hub 112, such that the needle guard 118 cannot be actuated to have the fingers 122 slide proximally into/within the slots 116, to move the needle guard 118 to its retracted position. When the needle guard 118 is in the unlocked position, the pair of fingers 122 are aligned with the pair of slots 116, such that the needle guard 118 may be actuated to have the fingers 122 slide proximally into/within the slots 116, to move the needle guard 118 to its retracted position.

In some embodiments, each of the port 24 and the needle guard 118 includes markings 130 thereon to identify a rotational position of the needle guard 118 relative to the hub 112 and port 24. When the markings 130 on the needle guard 118 are not aligned with the markings on the port 24, it is visible to an operator that the needle guard 118 is in the locked position. Conversely, when the markings 130 on the needle guard 118 are aligned with the markings 130 on the port 24, it is visible to an operator that the needle guard 118 is in the unlocked position. In some embodiments, the markings 130 on the port 24 and the needle guard 118 are provided as arrows.

Referring now to FIGS. 17-22, operation of the injection device 110 is described in more detail. In an initial configuration of the injection device 110, as shown in FIG. 17, the needle guard 118 is in an advanced position, where the needle guard 118 covers the distal end 38 of the needle 20. In the initial configuration, the needle guard 118 is oriented/rotated relative to the hub 112 so as to be in a locked position.

When it is desired to use the injection device 110 to administer the medicament to a patient, a user will first push the needle guard 118 proximally, in order to place the needle 20 in fluid communication with the reservoir 14, as shown in FIG. 18. When the needle guard 118 is pushed back proximally, the proximal end of fingers 122 on the needle guard 118 push against the hub 112, transferring force thereto. Further pushing of the needle guard 118 in the proximal direction causes the hub 112 to move proximally within port 24, such that the proximal end 36 of the needle 20 is driven/advanced through the pierceable membrane 34, so as to place the needle 20 in fluid communication with the reservoir 14.

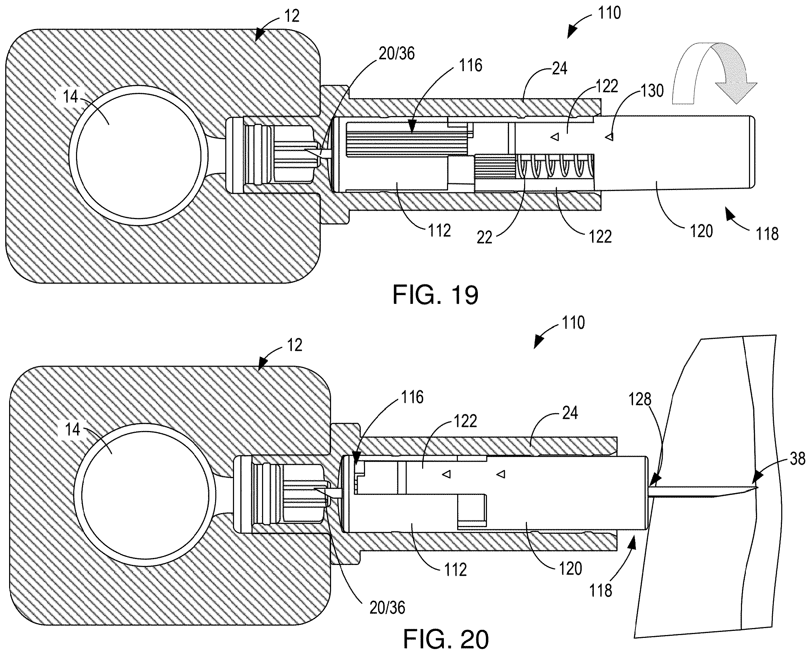

As shown in FIG. 19, upon placing of the needle 20 in fluid communication with the reservoir 14, the needle guard 118 may be rotated from the locked position to the unlocked position, in order to prepare for injection of the medicament into a patient. When rotated to the unlocked position, the pair of fingers 122 on the needle guard 118 are aligned with the pair of slots 116 in hub 112, as previously described.

With the needle guard 118 in the unlocked position, a user may position the injection device 110 adjacent the skin of the patient and apply a pushing force thereto. When the needle guard 118 is in the unlocked position and a proximally directed pushing force is applied to the needle guard 118 (by contact with the skin) that is greater than a biasing force of the biasing member 22, the needle guard 118 is moved from the advanced position to the retracted position, as shown in FIG. 20, with the pair of fingers 122 on the needle guard 118 sliding proximally into the pair of slots 116 in hub 112. Upon insertion of the distal end 38 of the needle 20 into the patient, the bulb-shaped reservoir 14 may be depressed (on both sides thereof) by a user, to force the medicament out from the reservoir 14 and through the needle 20, for administering to the patient.

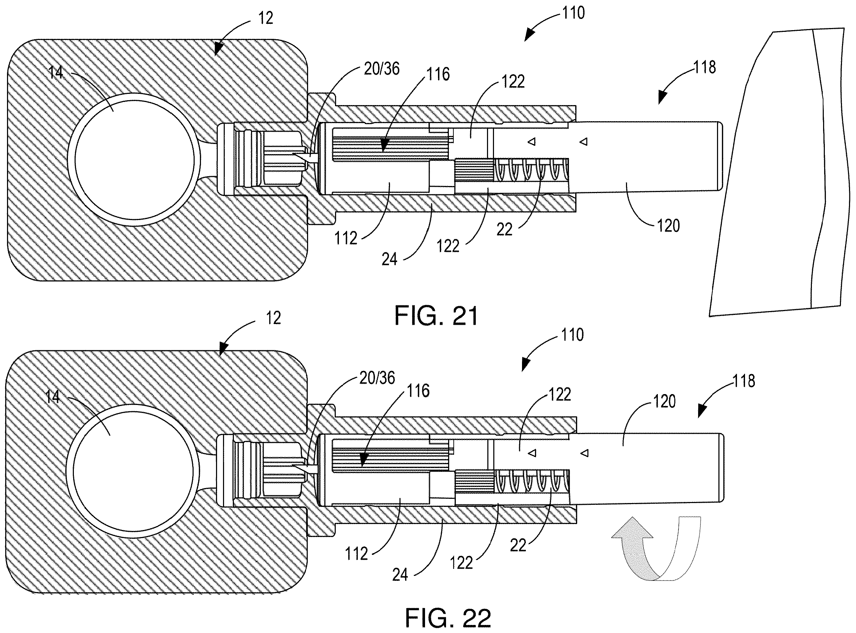

With the medicament administered to the patient, the user may proximally withdraw the injection device 110 (and the needle 20 thereof) away from the patient. As shown in FIG. 21, as the injection device 110 is withdrawn away from the patient, the biasing member 22 forces the needle guard 118 distally from the retracted position and toward the advanced position - with the fingers 122 of the needle guard 118 sliding within the slots 116 of hub 112. With the needle guard 118 moved to the advanced position, the distal end 38 of the double-ended needle 20 is again covered by the needle guard 118.

Upon the needle guard 118 reaching the advanced position, the user may then rotate the needle guard 118 from the unlocked position back to the locked position, as shown in FIG. 22. Rotation of the needle guard 118 back to its locked position prevents any subsequent unintended actuation of the needle guard 118 from its advanced position back to its retracted, thereby preventing any potential needle stick from occurring.

Beneficially, aspects of the disclosure described herein provide a single-use injection device with features for automatically disabling repeated use of the device and for shielding a needle of the device post-injection. A shield assembly is provided on the injection device that is movable from a retracted position to an advanced position, with the distal end of the needle extending out distally past the shield assembly when in the retracted position and the distal end of the needle covered by the shield assembly when in the advanced position. The shield assembly may automatically actuate to the advanced position after the injection is performed, or may be triggered by a user to actuate to the advanced position after the injection is performed.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A pre-fillable injection device comprising:
an end member graspable by a user and having a distal end and a proximal end, the end member comprising a film;
a reservoir defined within the end member and pre-filled with a fluid, wherein the reservoir is at least partially formed by the film, with the film being depressible by the user to reduce a volume of the reservoir to force the fluid out therefrom;
a port provided on the end member at the distal end thereof and configured to provide access to the reservoir, the port including a hub positioned therein;
a double-ended needle retained by the hub and having a proximal end positioned within the port and a distal end extending out from the port, the double-ended needle movable from a first position where the proximal end is separated from the reservoir to a second position where the proximal end is positioned to pierce into the reservoir, to place the needle in fluid communication with the reservoir;
a shield assembly positioned at the distal end of the end member and movable distally from a retracted position to an advanced position, with the distal end of the needle extending out distally past the shield assembly when in the retracted position and the distal end of the needle covered by the shield assembly when in the advanced position; and
a biasing member configured to actuate the shield assembly from the retracted position to the advanced position.

2. The pre-fillable injection device of claim 1, further comprising a removable needle cap, the removable needle cap depressible in a proximal direction to actuate the double-ended needle from the first position to the second position.

3. The pre-fillable injection device of claim 2, wherein the removable needle cap is removed after actuation of the double-ended needle from the first position to the second position, to enable insertion of the distal end of the needle into a patient.

4. The pre-fillable injection device of claim 2 or claim 3, wherein the port comprises a safety port including:
a port body defining a cavity and having an open distal end and a proximal end with an end cover thereat, with the port body having a pair of slots formed therein extending longitudinally along the port body and an opening formed in the port body on a side thereof, at a location between the pair of slots; and
a coupling member positioned at a proximal end of the port body and configured to couple the safety port to the end member.

5. The pre-fillable injection device of claim 4, wherein the shield assembly comprises:
a needle chamber positionable within the cavity and comprising:
a cylindrical body having an open proximal end, with the cylindrical body having a plurality of slots formed therein extending longitudinally along the cylindrical body and an annular flange formed at a distal end thereof, the annular flange extending radially inward and forming a seat; and
a cantilevered flexible tab coupled to a distal end of the cylindrical body, the flexible tab having a button thereon sized to be received within the opening of the port body; and
a pair of side members configured to movably couple the needle chamber to the safety port, each of the pair of side member comprising pins and locking tabs that engage with the pair of slots of the port body and the plurality of slots of the cylindrical body, to enable a longitudinal movement of the pair of side members and the needle chamber relative to the safety port;
wherein the biasing member is positioned between the safety port and the needle chamber and retained by the end cover and the seat, the biasing configured to translate the needle chamber distally out from the safety port, thereby transitioning the shield assembly from the retracted position to the advanced position.

6. The pre-fillable injection device of claim 5, wherein when the flexible tab is depressed inwardly, the button is disengaged from the opening to unlock the needle chamber from the safety port, thereby releasing the biasing member and enabling the distal movement of the needle chamber distally out from the safety port.

7. The pre-fillable injection device of claim 5 or claim 6, wherein engagement of the pins with the pair of slots of the port body and the plurality of slots of the cylindrical body limit the distal movement of the needle chamber distally out from the safety port, to prevent over-sliding.

8. The pre-fillable injection device of any of claims 5-7, wherein upon transitioning of the shield assembly to the advanced position, the locking tabs lock the needle chamber in place and prevent a transition back to the retracted position.

9. The pre-fillable injection device of any of claims 5-8, wherein the removable needle cap is coupled to the distal end of the cylindrical body of the needle chamber.

10. The pre-fillable injection device of claim 2 or claim 3, further comprising a guide member coupled to the port, the guide member including:
an elongated frame attached to the port and extending across a width of the end member, the elongated frame including a first through-hole positioned adjacent a first edge of the elongated frame, a second through-hole positioned adjacent a second edge of the elongated frame, and a third through-hole centered on the elongated frame and aligned with the port, with a seat formed about the third through-hole on a distal-facing side of the elongated frame; and
a pair of release tabs formed at opposing sides of the elongated frame and adjacent to the first and second through-holes.

11. The pre-fillable injection device of claim 10, wherein the shield assembly comprises a U-shaped slide member including:
a cross-bracket having a first end and a second end;
a first sliding guide positioned at the first end of the cross-bracket and extending proximally from the cross-bracket, the first sliding guide configured to slide through the first through-hole;
a second sliding guide positioned at the second end of the cross-bracket and extending proximally from the cross-bracket, the second sliding guide configured to slide through the second through-hole;
a receptacle centered on the cross-bracket; and
a safety chamber positioned within the receptacle, the safety chamber having an open proximal end and an open distal end;
wherein the biasing member is positioned between the guide member and the U-shaped slide member and retained by the seat and the receptacle, the biasing member configured to translate the U-shaped slide member distally away from the guide member, thereby transitioning the shield assembly from the retracted position to the advanced position.

12. The pre-fillable injection device of claim 11, wherein each of the first sliding guide and the second sliding guide comprises:
a pair of distal support tabs; and
a pair of proximal support tabs;
wherein each of the pair of distal support tabs is engageable with a notch formed a respective one of the first through-hole and the second through-hole; and
wherein the pair of proximal support tabs are engageable with proximal- and distal-facing sides of the elongated frame.

13. The pre-fillable injection device of claim 12, wherein with the shield assembly in the retracted position, one of the pair of distal support tabs on each of the first and second sliding guides is engaged with the notch in the respective one of the first through-hole and the second through-hole, to lock the U-shaped slide member in the retracted position.

14. The pre-fillable injection device of claim 13, wherein when the pair of release tabs are depressed inwardly, the one of the pair of distal support tabs on each of the first and second sliding guides button is disengaged from the notch to unlock the U-shaped slide member from the guide member, thereby releasing the biasing member and enabling the distal movement of the U-shaped slide member away from the guide member.

15. The pre-fillable injection device of any of claims 12-14, wherein with the shield assembly in the advanced position, the pair of proximal support tabs engage the proximal- and distal-facing sides of the elongated frame, to lock the U-shaped slide member in place and prevent a transition back to the retracted position.

16. The pre-fillable injection device of any of claims 11-15, wherein with the shield assembly in the advanced position, the distal end of the double-ended needle is positioned within the safety chamber.

17. The pre-fillable injection device of any of claims 12-16, wherein the shield assembly further comprises a flexible hook coupled to an inner surface of the safety chamber, the flexible hook positioned alongside the needle and deflected thereby when the shield assembly is in the retracted position and positioned in an un-deflected position that covers the distal end of the needle when the shield assembly is in the advanced position.

18. The pre-fillable injection device of claim 1, wherein the hub comprises a pair of slots formed therein extending longitudinally along the hub.

19. The pre-fillable injection device of claim 18, wherein the shield assembly comprises a needle guard coupled to the hub, the needle guard comprising:
a cylindrical cover portion having a closed distal end, the closed distal end including a needle hole formed therein through which the needle may pass; and
a pair of fingers extending out proximally from the cylindrical cover portion and configured to engage and slide within the pair of slots;
wherein the needle guard is rotatable between a locked position that prevents proximal movement of the needle guard relative to the hub and an unlocked position that allows proximal movement of the needle guard relative to the hub, with the pair of fingers misaligned with the pair of slots when in the locked position and aligned with the pair of slots when in the unlocked position.

20. The pre-fillable injection device of claim 19, wherein each of the port and the needle guard includes markings thereon to identify a rotational position of the needle guard as either in the locked position or the unlocked position.

21. The pre-fillable injection device of any of claims 19 or claim 20, wherein the biasing member is positioned between a distal end of the hub and the closed distal end of the needle guard, with the biasing member configured to apply a biasing force to the needle guard that biases the needle guard to the advanced position.

22. The pre-fillable injection device of claim 21, wherein when the needle guard is in the unlocked position and a proximally directed pushing force is applied to the needle guard that is greater than the biasing force, the needle guard is moved from the advanced position to the retracted position, with the pair of fingers sliding proximally into the pair of slots.

23. The pre-fillable injection device of any of claims 19-22, wherein when the needle guard is in the locked position, the needle guard is depressible in a proximal direction to actuate the double-ended needle from the first position to the second position.

24. The pre-fillable injection device of any of claims 1-23, wherein the biasing member comprises a spring configured to transition from a compressed state to an extended state, the spring transitioning from the compressed state to the extended state to move the shield assembly from the retracted position to the advanced position.
